# EUROPÄISCHE PATENTSCHRIFT

(11) **EP 2 620 108 B1**
(45) Veröffentlichungstag und Bekanntmachung des Hinweises auf die Patenterteilung: **03.05.2017**
(21) Anmeldenummer: 12193362.6
(22) Anmeldetag: 20.11.2012
(51) Int. Cl.: A61B 17/16, A61B 17/88

(54) **Knochenfräser, Sortiment sowie System mit Knochenfräser**
Bone milling tool, assorted set and system with bone milling tool
Fraise à os, assortiment et système avec fraise à os

(30) Priorität: 24.01.2012 DE 102012100565
(43) Veröffentlichungstag der Anmeldung: 31.07.2013
(73) Patentinhaber: Zimmer GmbH, 8404 Winterthur (CH)
(72) Erfinder: Pech, Uwe, 78532 Tuttlingen (DE)
(74) Vertreter: Manitz Finsterwald Patentanwälte PartmbB

(56) Entgegenhaltungen:
- WO-A1-02/49516
- US-A- 4 023 572
- US-A- 5 755 719

## Beschreibung

Die Erfindung betrifft einen chirurgischen Knochenfräser, insbesondere einen sogenannten Cup- and Conefräser, gemäß dem Oberbegriff des Anspruchs 1, insbesondere für die Arthrodese eines, bevorzugt des ersten, Metatarsophalangealgelenks, mit einem rotierbaren Fräsmesserhalter und mindestens einem Fräsmesser zur Herstellung einer konkaven oder konvexen Fräskontur im Knochen, welches mit Fixiermitteln lösbar am Fräsmesserhalter fixierbar ist. Ferner betrifft die Erfindung ein Sortiment mit einem solchen Knochenfräser gemäß Anspruch 8 und ein System mit einem Knochenfräser und einem Lösewerkzeug gemäß Anspruch 9. Gattungsgemäße chirurgische Knochenfräser, sogenannte Cup- & Conefräser sind seit langem bekannt. Diese werden in der Fußchirurgie in erster Linie bei der Arthrodese des ersten Metatarsophalangealgelenks eingesetzt. Dabei wird zunächst ein erstes Knochenteil konkav und ein zweites Knochenteil korrespondierend konvex gefräst, woraufhin dann die Knochenteile in der Art einer Kugel-Pfannenverbindung zusammengefügt und dann fixiert werden, so dass erstes und zweites Knochenteil zusammenwachsen.
Die auf dem Markt erhältlichen chirurgischen (Fuß-) Knochenfräser sind in der Regel einteilig, d.h. aus einem Guss bzw. an einem Stück ausgebildet. Neben diesen einteiligen chirurgischen Knochenfräsern sind Knochenfräser bekannt geworden, bei denen das Fräsmesser austauschbar ist, beispielsweise um dieses erleichtert schärfen zu können. Einen derartigen chirurgischen Knochenfräser beschreibt die US 4,621,637. Der bekannte Knochenfräser weist einen Fräsmesserhalter auf, der mit einem entsprechenden Elektrowerkzeug antreibbar ist.

In stirnseitigen Öffnungen des Fräsmesserhalters sind zwei Fräsmesser einsetzbar, wobei die Fixierung, d.h. axiale Sicherung der Fräsmesser am Fräsmesserhalter über ein Kuppelteil erfolgt, welches über Elastomerringe in entsprechenden Öffnungen im Fräsmesserhalter fixierbar ist. Nachteilig bei dem bekannten chirurgischen Knochenfräser ist dessen komplexer Aufbau und die große Anzahl an benötigten Einzelteilen, was eine Montage und Demontage und somit auch die Reinigbarkeit erschwert.

Ein alternativer chirurgischer Knochenfräser mit demontierbarem Fräsmesser ist aus der US 3,630,204 bekannt. Zum axialen Sichern des Fräsmessers ist eine Fixierschraube vorgesehen, die seitlich in einem Innengewindeloch des Fräsmesserhalters eindrehbar ist und eine Fixieröffnung des Fräsmessers durchsetzt. Auch hier ist die Montage und Demontage schwierig bzw. zeitaufwändig, da es notwendig ist zum Festlegen bzw. Lösen eine Fixierschraube fest- bzw. aufzuschrauben. Darüber hinaus ist die Reinigbarkeit verbesserungsbedürftig.

Aus der US 4,023,572 ist ein Knochenfräser bekannt, bei dem ein Fräsmesser über Federkraft gehalten wird. Die Druckschrift beschreibt unterschiedliche Möglichkeiten zur mittelbaren federnden Fixierung, beispielsweise über axial federnd gelagerte Stifte oder federnd gelagerte Haken.

Die WO 02/49516 A1 zeigt einen Knochenfräser, bei dem ein Fräsmesser über eine federgelagerte Kugel fixiert wird.

Zum weiteren Stand der Technik wird die US 5,755,719 A genannt.

Ausgehend von dem vorgenannten Stand der Technik liegt der Erfindung die Aufgabe zugrunde, einen verbesserten chirurgischen Knochenfräser mit einem austauschbaren Fräsmesser anzugeben, wobei sich der Knochenfräser durch einen konstruktiv einfachen Aufbau, sowie vor allem durch eine einfache Montier- und Demontierbarkeit auszeichnen soll. Darüber hinaus soll der Knochenfräser möglichst wenig Einzelteile aufweisen und gut reinigbar sein. Ferner besteht die Aufgabe darin, ein eine hohe Flexibilität ermöglichendes Sortiment mit einem derartig verbesserten Knochenfräser anzugeben sowie ein System aus einem Knochenfräser und einem Lösewerkzeug, das eine einfache und schnelle Zerlegbarkeit des Knochenfräsers bzw. ein einfaches Austauschen des Fräsmessers auszeichnet.

Diese Aufgabe wird hinsichtlich des, vorzugsweise aus Titan und/oder Chirurgenstahl ausgebildeten, Knochenfräsers mit den Merkmalen des Anspruchs 1, hinsichtlich des Sortimentes mit den Merkmalen des Anspruchs 8 und hinsichtlich des Systems mit den Merkmalen des Anspruchs 9 gelöst.

Vorteilhafte Weiterbildungen der Erfindung sind in den Unteransprüchen angegeben. In den Rahmen der Erfindung fallen sämtliche Kombinationen aus zumindest zwei von in der Beschreibung, den Ansprüchen und/oder den Figuren offenbarten Merkmalen.

Der Erfindung liegt der Gedanke zugrunde, das Fräsmesser unmittelbar mit Hilfe von Rastmitteln am Fräsmesserhalter festzulegen, wobei es besonders bevorzugt ist, wenn auf zusätzliche Fixiermittel, insbesondere auf Schrauben oder abnehmbare Halterungen, wie beispielsweise eine in der US 4,621,637 beschriebene Haltekuppel verzichtet wird. Die unmittelbare Verrastung des Fräsmessers über ein Federelement bringt erhebliche Vorteile mit sich. So reduziert sich die Teilezahl auf ein Minimum; neben mindestens einem Fräsmesser und einem Fräsmesserhalter wird bevorzugt nur ein Federelement, ggf. auch mehrere Federelemente benötigt. Hier wird nicht nur ein konstruktiv einfacher Aufbau gewährleistet - es resultiert auch unmittelbar eine gute Reinigbarkeit. Bevorzugt ist der erfindungsgemäße Knochenfräser als Handknochenfräßer oder als Fußknochenfräser für die Arthrodese eines, insbesondere des ersten, Metatarsophalangealgelenks, ausgebildet. Je nach Ausgestaltung des Fräsmessers können mit dem Knochenfräser auch Gelenkflächen bearbeitet bzw. erneuert werden.

Besonders zweckmäßig ist eine Ausführung des Knochenfräsers, bei der das mindestens eine, vorzugsweise ausschließlich eine, Federelement derart ausgebildet und angeordnet ist, dass dieses beim Festlegevorgang des Fräsmessers in radialer Richtung spannbar, d.h. entgegen der Federkraft des Federelementes bewegbar ist und im verrasteten Zustand mit zumindest reduzierter Spannung oder ohne Spannung in einer Rastausnehmung aufgenommen ist. Je nach Anordnung des Federelementes kann die, vorzugsweise als mindestens eine Rastnut ausgebildete Rastausnehmung am Fräsmesser oder am Fräsmesserhalter angeordnet sein. Bevorzugt wird das Federelement auch bei dem Lösen aus der Verraststellung zeitweise gespannt, wobei besonders bevorzugt sowohl beim Festlegen als auch beim Lösen die Federspannung des Federelementes überwunden werden muss. Besonders zweckmäßig ist es, wenn das Federelement das Fräsmesser und/oder den Fräsmesserhalter während des Montage- und/oder Demontagevorgangs in radialer Richtung nach innen oder außen federkraftbeaufschlagt.

Im Hinblick auf die Anordnung bzw. Positionierung des Federelementes gibt es unterschiedliche Möglichkeiten. Gemäß einer ersten, bevorzugten Variante ist das Federelement, insbesondere in einer Nut, noch weiter bevorzugt einer Umfangsnut des Fräsmesserhalters festgelegt und wird beim Festlegen des Fräsmessers durch unmittelbare Wechselwirkung mit dem Fräsmesser gespannt, wobei das Federelement in der Verrastposition vorzugsweise zumindest weniger gespannt ist als zu einem Zeitpunkt während des Festlegevorgangs. Ganz besonders bevorzugt ist es hierbei, wenn das Fräsmesser das Federelement beim Festlegen und/oder Lösen in radialer Richtung nach außen federkraftbeaufschlagt, wobei auch eine Ausführungsform realisierbar ist, bei der die Kraftbeaufschlagung in radialer Richtung nach innen erfolgt.

Bei einer weiteren Variante ist das Federelement am Fräsmesser festgelegt und wird durch unmittelbare Wechselwirkung mit dem Fräsmesserhalter gespannt, vorzugsweise dann in radialer Richtung nach innen, wobei auch alternativ eine Spannung in radialer Richtung nach außen möglich ist. Zudem ist es denkbar, beide vorerwähnten Varianten (mit zwei unterschiedlichen Federelementen) gemeinsam zu realisieren.

Besonders bevorzugt ist eine Ausführungsform, bei welcher das Fräsmesser und/oder der Fräsmesserhalter das Federelement im verrasteten Zustand axial innen durchsetzt/durchsetzen oder außen umgreifen.

Gemäß einer besonders bevorzugten Ausführungsvariante ist als, insbesondere einziges, Federelement mindestens ein, bevorzugt metallischer Federring vorgesehen, der noch weiter bevorzugt seine Federwirkung aufgrund einer geschlitzten Ausführung entfaltet. Anstelle des Vorsehens eines federnd aufweitbaren und/oder komprimierbaren Federrings als Federelement ist es denkbar, mindestens zwei, bevorzugt in Umfangsrichtung beabstandete, beispielsweise teilringförmige Federn vorzusehen, die noch weiter bevorzugt in radialer Richtung nach innen und/oder außen während des Festlegens und/oder Lösens des Fräsmessers spannbar sind.

Um die Montage des Fräsmessers am Fräsmesserhalter weiter zu vereinfachen, ist in Weiterbildung der Erfindung mit Vorteil vorgesehen, dass zum radialen Spannen des Fräsmessers während des Festlegens - je nach Anordnung des Federelementes am Fräsmesserhalter oder am Fräsmesser, am Fräsmesser bzw. am Fräsmesserhalter eine Anlaufschräge vorgesehen ist, mit der das Federelement, insbesondere in radialer Richtung nach außen oder innen bei einer axialen Relativbewegung mit einer Kraft, insbesondere einer Radialkraft beaufschlagbar und dadurch spannbar ist, um dann nach Überwindung der Anlaufschräge in der Rastausnehmung aufgenommen zu werden. Um ein unbeabsichtigtes Lösen des Fräsmessers sicher zu verhindern, ist es besonders zweckmäßig, wenn zum Lösen des Fräsmessers keine solche Anlaufschräge vorgesehen ist, oder eine weniger steile, d.h. schwere überwindbare Anlaufschräge.

Als besonders zweckmäßig hat es sich herausgestellt, wenn an dem Fräsmesserhalter, insbesondere eine seitliche, Werkzeugaufnahme (Öffnung) zur Aufnahme eines Lösewerkzeugs vorgesehen ist, wobei das Fräsmesser, insbesondere axial in diese Aufnahme hineinragt, so dass das Fräsmesser in der Aufnahme in axialer Richtung in eine Löserichtung von dem Fräsmesserhalter weg mittels des Lösewerkzeugs kraftbeaufschlagbar ist. Dies kann beispielsweise dadurch realisiert werden, dass am Lösewerkzeug eine Anlaufschräge vorgesehen ist, die durch Einschieben des Lösewerkzeugs in die Aufnahme in Wechselwirkung mit dem Fräsmesser tritt und dieses nach oben schiebt. Alternativ ist es denkbar Lösewerkzeug und Aufnahme derart aufeinander abzustimmen, dass das Lösewerkzeug, insbesondere spannungsfrei in die Aufnahme einführbar und dort verdrehbar ist und dadurch das Fräsmesser in axialer Richtung lösekraftbeaufschlagt.

Besonders zweckmäßig ist es, wenn das Federelement ausschließlich die Aufgabe hat bzw. so ausgebildet ist, dass es das Fräsmesser gegen axiales Lösen sichert. Bevorzugt sind zusätzlich Formschlussmittel vorgesehen, die derart mit dem Fräsmesser zusammenwirken bzw. auf dieses abgestimmt sind, dass ein Verdrehen des Fräsmessers relativ zu dem Fräsmesserhalter in Umfangsrichtung sicher vermieden wird. Im einfachsten Fall werden die Formschlussmittel von einer Axialführung für das Fräsmesser gebildet, in welche das, wie später noch erläutert werden wird, bevorzugt in einer axialen Projektion kreuzförmige Fräsmesser eingesetzt werden kann. Im Falle eines konkaven Fräsmessers ist es bevorzugt, wenn dieses in einem radial äußeren Bereich in Umfangsrichtung gesichert wird, beispielsweise durch radiale Schlitze in einer Umfangswand des Fräsmesserhalters. Bei dem konvexem Fräsmesser ist es bevorzugt, wenn die Führung bzw. Verdrehsicherung in einem radial unteren Bereich angeordnet ist.

Als besonders zweckmäßig hat es sich herausgestellt, wenn das, insbesondere als Federring ausgebildete Federelement die vorerwähnte Verdrehsicherung, die bevorzugt als Axialführung ausgebildet ist, in Umfangsrichtung durchsetzt, so dass das Federelement im Bereich der Drehsicherung mit dem Fräsmesser oder dem Federhalter verrastet.

Im Hinblick auf die Ausgestaltung des Fräsmessers gibt es unterschiedliche Möglichkeiten. Zum Erzielen eines optimalen Fräsergebnisses, hat es sich als besonders vorteilhaft herausgestellt, wenn ein sich in der axialen Projektion kreuzförmiges Fräsmesser eingesetzt wird. Das Fräsmesser kann einteilig ausgebildet sein oder aus mehreren, insbesondere zwei, noch weiter bevorzugt axial ineinandersteckbaren Teilen bestehen. Auch ist es möglich zwei Messerteile, insbesondere Messerscheiben dauerhaft fest durch Verkleben oder Verschweißen miteinander zu verbinden. Im Hinblick auf das Fräsergebnis und die resultierende Oberflächenstruktur hat es sich weiterhin als vorteilhaft erwiesen, die Fräsmesserschneide, zumindest abschnittsweise, vorzugsweise vollständig in Form eines Wellenschliffes auszubilden, da entsprechend unebene Knochenstrukturen ein Zusammenwachsen fördern, wobei gemäß einer ersten Variante insgesamt ein symmetrischer Wellenschliff gewählt wird, bei welchem Wellenberge von zwei in Umfangsrichtung voneinander beabstandeten Messerabschnitten auf dem jeweils gleichen Radius liegen, ebenso wie die jeweils dazu benachbarten Wellentäler. Gemäß einer zweiten Variante wird ein asymmetrischer Schliff gewählt, um die Unebenheiten im Knochen zu minimieren. Bei einem asymmetrischen Wellenschliff liegen in Umfangsrichtung benachbarte Wellenberge nicht auf demselben Radius, sondern sind radial versetzt angeordnet, wobei es besonders bevorzugt ist, wenn mindestens ein Wellenberg eines ersten Messerabschnitts auf demselben Radius liegt, wie ein in Umfangsrichtung beabstandetes Wellental.

Herkömmliche Knochenfräser weisen häufig eine Öffnung zur Aufnahme eines Kirschnerdrahtes auf, wobei hierdurch die Aussetzrichtung exakt vordefiniert wird. In Weiterbildung der Erfindung ist anstelle einer solchen Kirschnerdrahtöffnung eine, insbesondere kleine Zentrierspitze vorgesehen, wodurch der Operateur beim Auffräsen der Knochenflächen wesentlich flexibler ist - dieser kann mit horizontal rotierenden Bewegungen eingesetzt werden, so dass exakte Knochenflächen in einem Arbeitsgang gefräst werden können.

Die Erfindung führt auch auf ein Sortiment, umfassend mindestens einen erfindungsgemäßen Knochenfräser, wobei das Sortiment neben mindestens einem Fräsmesserhalter mindestens zwei mit dem Fräsmesserhalter verwendbare Fräsmesser umfasst, die unterschiedlich ausgebildet sind, insbesondere um unterschiedlich große konvexe oder konkave Fräsungen durchzuführen. Insbesondere umfasst das Sortiment mindestens zwei, in ihrem Fräsdurchmesser unterschiedliche Konkavmesser oder mindestens zwei unterschiedliche Konvexmesser. Somit ist es möglich, mit ein und demselben Fräsmesserhalter mindestens zwei unterschiedliche, vorzugsweise mehr als zwei unterschiedliche Fräsmesser zu verwenden. Die unterschiedlichen Fräsmesser eines Sortimentes sind im Hinblick auf die Schnittstelle mit dem Fräsmesserhalter identisch ausgebildet und unterscheiden sich bevorzugt nur in der Schneidengeometrie.

Ganz besonders bevorzugt umfasst das Sortiment mindestens zwei unterschiedliche Konkavmesser oder Konvexmesser aus der folgenden Messergruppe mit einem maximalen Klingen-Durchmesser von 12mm, 14mm, 16mm, 19mm, 21 mm und/oder 23mm.

Ferner führt die Erfindung auch auf ein System, umfassend einen nach dem Konzept der Erfindung ausgebildeten Knochenfräser sowie ein Lösewerkzeug, das in eine, insbesondere seitliche, Aufnahme des Fräsmessers einführbar ist, wobei das Fräsmesser im mit dem Fräsmesserhalter verrasteten Zustand derart in die Aufnahme hineinragt, insbesondere senkrecht zu einer Aufnahmelängserstreckung, dass das Fräsmesser mittels des Lösewerkzeugs, insbesondere durch Einschieben und/oder Verdrehen des Lösewerkzeugs in die bzw. in der Aufnahme derart axial in einer Löserichtung kraftbeaufschlagbar ist, dass das Fräswerkzeug das Federelement in radialer Richtung spannt, um aus der Verrastung freizukommen. Bei der Realisierung eines Lösens durch Verdrehen ist es bevorzugt, wenn die Aufnahme eine kreisförmige Umfangskontur aufweist und das Lösewerkzeug eine entsprechende Teilkreiskontur mit einem abgeflachten Abschnitt, der ein Schieben unter das Fräsmesser ermöglicht, wobei durch Verdrehen des Lösewerkzeugs der Teilkreisabschnitt in Kontakt mit dem Fräsmesser kommt, und dieses axial kraftbeaufschlagt.

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:

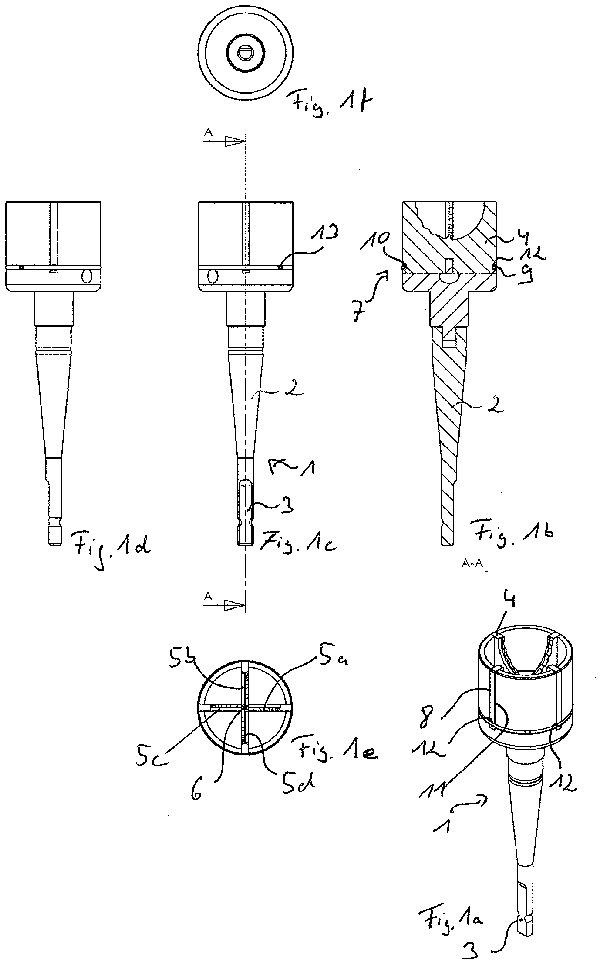

- Fig. 1 a:: eine perspektivische Schrägansicht eines montierten chirurgischen (Fuß-) Knochenfräsers mit konkavem Fräsmesser bzw. mit einem Fräsmesser zur Herstellung einer konvexen Fräsung,
- Fig. 1 b bis Fig. 1 f:: unterschiedliche, teilweise geschnittene zweidimensionale Ansichten des Knochenfräsers gemäß Fig. 1 a,
- Fig. 2a bis Fig. 2g:: unterschiedliche Ansichten des konkaven Fräsmessers für einen Knochenfräser gemäß den Fig. 1a bis 1f, wobei zur Verdeutlichung das an dem in den Fig. 2a bis 2g nicht dargestellten Fräsmesserhalter festgelegte Federelement im verrasteten Zustand dargestellt ist,
- Fig. 3a:: eine perspektivische Ansicht eines alternativen (Fuß-) chirurgischen Knochenfräsers mit konvexem Fräsmesser zur Herstellung konkaver Fräsungen,
- Fig. 3b bis Fig. 3e:: unterschiedliche, teilweise geschnittene zweidimensionale Ansichten des Knochenfräsers gemäß Fig. 3a,
- Fig. 4a bis Fig. 4e:: unterschiedliche, teilweise geschnittene Ansichten eines konvexen Fräsmessers für einen Knochenfräser gemäß den Fig. 3a bis 3e, wobei auch hier analog zu den Fig. 2a bis 2e das am Fräsmesserhalter festgelegte Federelement in verrastetem Zustand dargestellt ist,
- Fig. 5 bis Fig. 8:: einzelne Schritte und Details des rastenden Festlegens eines Fräsmessers an einem Fräsmesserhalter,
- Fig. 9 bis Fig. 16:: einzelne Schritte und Details der Demontage des Fräsmessers vom Fräsmesserhalter,
- Fig. 17 bis Fig. 22:: unterschiedliche Einzelteile und Details von Ausführungen eines konvexen Fräsmessers zur Herstellung von konkaven Fräsungen, und
- Fig. 23 bis Fig. 28:: unterschiedliche Einzelteile und Ausführungen eines konkaven Fräsmessers zur Herstellung konvexer Fräsungen.

In den Figuren sind gleiche Elemente und Elemente mit der gleichen Funktion mit den gleichen Bezugszeichen gekennzeichnet.

In den Fig. 1a bis 1f ist ein erstes Ausführungsbeispiel eines chirurgischen Knochenfräsers 1 gezeigt. Dieser umfasst einen mittels eines Elektrowerkzeugs rotierbaren Fräsmesserhalter 2, der im Bereich seines hinteren bzw. in der Zeichnung unteren Endes einen Antrieb 3 zum Zusammenwirken mit einem solchen Werkzeug aufweist. Der Fräsmittelhalter 2 trägt auf der von dem Antrieb 3 abgewandten Seite ein Fräsmesser 4, in dem konkreten Ausführungsbeispiel ein konkaves Fräsmesser 4 zur Herstellung konvexer Fräsungen.

Das Fräsmesser 4 weist vier um jeweils 90° zueinander versetzte Klingenabschnitte 5a bis 5d auf, in deren Zentrum sich eine Zentrierspitze 6 befindet.

Das Fräsmesser 4 ist mit Hilfe von später noch zu erläuternden Fixiermitteln 7 ausschließlich durch Verrasten in axialer Richtung gesichert bzw. am Fräsmesserhalter lösbar festgelegt. Als Verdrehsicherung sind Formschlussmittel 8 in Form von Axialführungen vorgesehen, die ein Verdrehen des Fräsmessers 4 relativ zu dem Fräsmesserhalter 2 sicher verhindern. Die Fixiermittel 7 umfassen ein, hier einziges, als Federring ausgebildetes Federelement 9, welches in einer (Außen-) Umfangsnut 10 des Fräsmesserhalters 2 aufgenommen ist. Das Federelement 9 durchsetzt in Umfangsrichtung Axialführungen 11 für das Fräsmesser 4 und liegt im Bereich dieser Axialführungen 11 im verrasteten Zustand in umfangsseitigen Rastausnehmungen 12 im Fräsmesser 4 und sichert dieses in axialer Richtung. Wie sich aus den Figuren 1a bis 1f ergibt, ist das als Federring ausgebildete Federelement 9 an einer Umfangsstelle mit einem Schlitz 13 versehen, d.h. unterbrochen und kann so federnd aufgeweitet werden.

Wie sich insbesondere aus der Schnittdarstellung gemäß Fig. 1b ergibt durchsetzt das Fräsmesser 4 das Federelement 9 im verrasteten Zustand in axialer Richtung und umgreift das Federelement 9 teilweise von radial innen her durch Aufnahme des Federelementes 9 in den vier um 90° zueinander versetzten Rastausnehmungen.

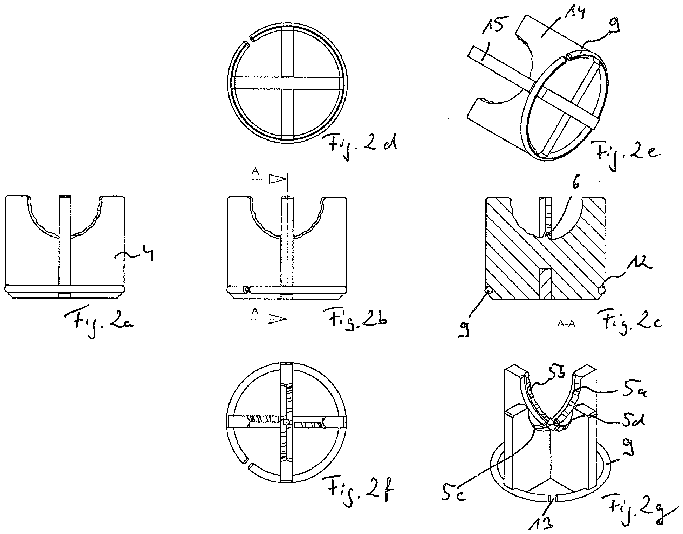

In den Fig. 2a bis 2g ist das Fräsmesser 4 des Knochenfräsers 1 gemäß den Fig. 1a bis 1f im Detail in unterschiedlichen Ansichten gezeigt, zusammen mit dem eigentlich am Fräsmesserhalter 2 festgelegten Federelement 9, welches in der verrasteten Stellung dargestellt ist, also aufgenommen in den vier Rastaufnehmungen 12 des Fräsmessers 4.

Zu erkennen ist, dass das Fräsmesser 4 aus einem ersten und einem zweiten Messerteil 14, 15 zusammengesetzt ist, wobei sich die Messerteile 14, 15 kreuzen. Die Messerteile 14, 15 können alternativ lösbar ineinander gesteckt oder dauerhaft fest, beispielsweise durch Verkleben oder Verschweißen miteinander verbunden sein. Theoretisch denkbar ist auch eine einteilige Ausführung des Fräsmessers 4.

Die Messerteile 14, 15 weisen in einem inneren Bereich die vier Klingenabschnitte 5a bis 5d auf und sind an ihrem Außenumfang gradlinig konturiert, in der Art von Plattenseiten. Mit diesen Plattenseiten können die Messerteile 14, 15 in diesen zugeordneten Axialführungen 11 durch axiales Einschieben aufgenommen werden, wobei die Axialführungen die Messerzeile 14, 15 in Umfangsrichtung umgeben, um so ein Verdrehen des Fräsmessers 4, relativ zu dem Fräsmesserhalter 2, sicher zu verhindern. Im Bereich dieser Axialführungen ist das Fräsmesser 4, d.h. an insgesamt vier Stellen mit dem als Ringfederelement ausgebildeten Federelement 9 verrastet.

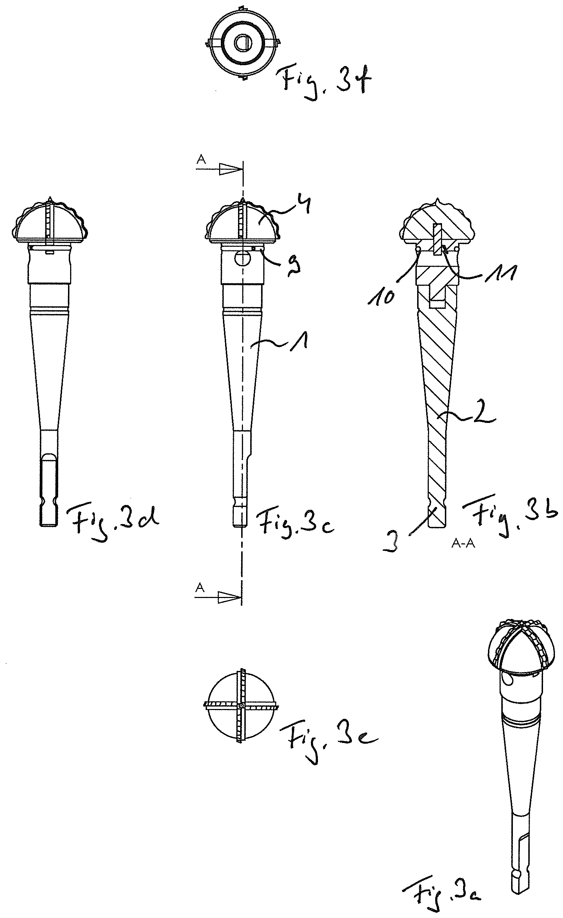

In den Fig. 3a bis 3f ist ein alternativer Knochenfräser 1 dargestellt, der nach einem ähnlichen Prinzip aufgebaut ist wie der Knochfräser gemäß den Fig. 1a bis 1f, so dass zur Vermeidung von Wiederholungen im Hinblick auf Gemeinsamkeiten auf die vorstehenden Figuren mit zugehöriger Beschreibung verwiesen wird. Im Folgenden wird im Wesentlichen auf Unterschiede eingegangen.

Zu erkennen ist, dass das Fräsmesser 4 als Konvexmesser zur Herstellung von konkaven Fräsungen ausgebildet ist. Auch bei der Ausführungsform gemäß den Fig. 3a bis 3f ist das als Ringfederelement ausgebildete Federelement 9 in einer entsprechenden Umfangsnut 10 des Fräsmittelhalters 2 aufgenommen und wird im verrasteten Zustand in axialer Richtung durchsetzt von dem Fräsmesser 4, welches in einem radial inneren Bereich axial in einer zentrischen Axialführung 11 geführt ist.

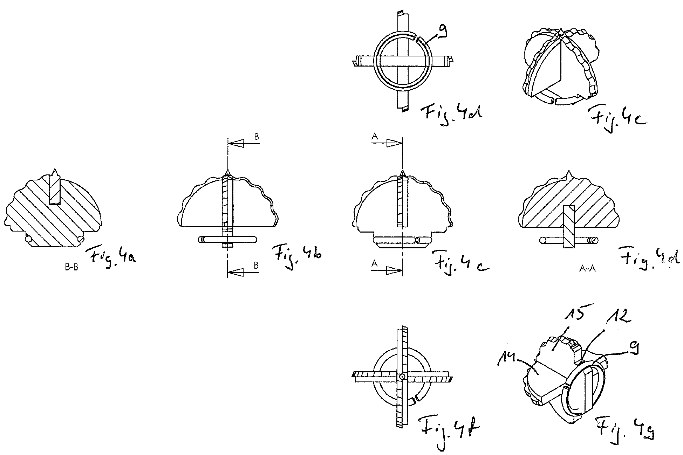

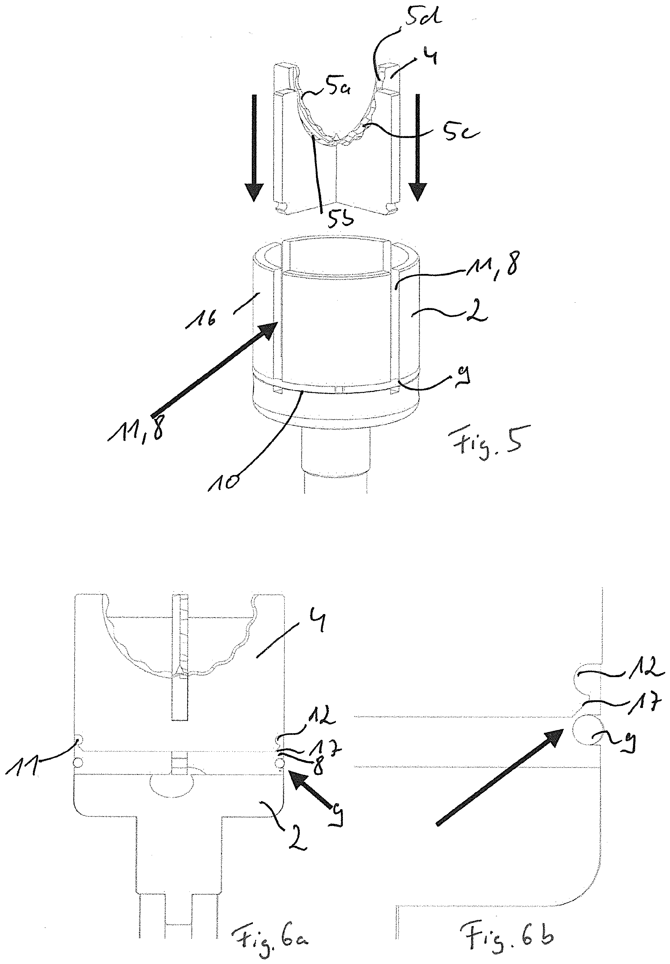

In den Fig. 4a bis 4g sind unterschiedliche Darstellungen des konvexen Fräsmessers 2 gezeigt, welches mit dem Federelement 9 verrastet ist, das hierzu in zwei diametral gegenüberliegenden umfangsseitigen Rastausnehmungen 12 aufgenommen ist. Das Federelement 9 ist im verrasteten Zustand axial durchsetzt von zwei Messerteilen 14, 15, die analog zu dem anderen Ausführungsbeispiel lösbar aneinander gesteckt oder dauerhaft fest miteinander verbunden sein können. In dem gezeigten Ausführungsbeispiel durchsetzt lediglich das zweite Messerteil 15 das Federelement und ist in Fräshalter 2 in der vorerwähnten Axialführung aufgenommen. Anhand der Fig. 5 bis 8 wird beispielhaft anhand eines Knochenfräsers mit konkavem Fräsmesser der Montagevorgang erläutert. Zu erkennen ist zunächst in Fig. 5 der Fräsmesserhalter 2 mit seinen als Axialführungen 11 ausgebildeten Formschlussmitteln 8 zur Verdrehsicherung des Fräsmessers 4, welches von oben her in durch zwei parallele Pfeile angedeuteter axialer Richtung auf den Fräsmesserhalter 2 zubewegt wird, und zwar derart, dass das in der axialen Projektion kreuzförmige Fräsmesser randseitig in den Axialführungen 11 aufgenommen werden. In Fig. 5 ist sehr deutlich zu erkennen, dass bei dem gezeigten Ausführungsbeispiel die Axialführungen 11 von linearen Unterbrechungen einer hohlzylindrischen Umfangswand 16 begrenzt sind, die die Klingenabschnitte 5a bis 5d in montiertem Zustand radial außen umgeben.

Aus Fig. 5 ergibt sich weiterhin, dass das als metallischer Federring ausgebildete Federelement 9 in der Umfangsnut 10 der Umfangswand 11 aufgenommen ist und die Axialführungen 11 in Umfangsrichtung durchsetzt.

Wie sich aus dem nächsten Schritt gemäß den Darstellungen gemäß den Fig. 6a und 6b ergibt, ist das Fräsmesser 4 mit insgesamt 4 Anlaufschrägen 17 versehen, die derart angeordnet sind, dass durch Axialkraftbeaufschlagung des Fräsmessers 4 eine Radialkraftkomponente auf das Federelement 9 resultiert, welches dieses beim Montagevorgang in radialer Richtung nach außen drückt und dadurch zeitweise spannt, bis die Anlaufschräge 17 überwunden ist und das Federelement 9 in radialer Richtung nach innen zurück in die Rastausnehmungen 12 im Bereich der Axialführungen 9 einschnappen kann.

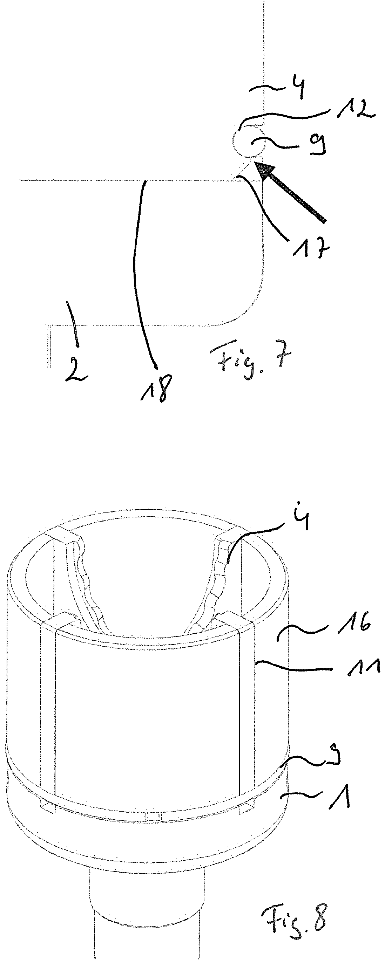

In Fig. 7 ist die Anlaufschräge 17 überwunden, das Fräsmesser 4 liegt axial auf einer Anlagefläche 18 des Fräsmesserhalters 2 auf und das Federelement 9 liegt, vorzugsweise dicht gespannt, jedenfalls weniger gespannt als während des Montagevorgangs bzw. während der Anlage des Federelementes 9, an den Anlaufschrägen 17 in den Rastausnehmungen 12.

Anders ausgedrückt untergreift das Fräsmesser 4 mit einer unteren Wand der Rastausnehmung 12 das Federelement 9, wodurch das Fräsmesser 4 gegen axiales Abheben von dem Fräsmesserhalter 2 gesichert ist. Fig. 8 zeigt ausschnittsweise den montierten Knochenfräser 1 mit dem Fräsmesser 4, welches in Umfangsrichtung in den Axialführungen 11 der Umfangswand 16 drehgesichert und von dem Federelement 9 in axialer Richtung gesichert ist.

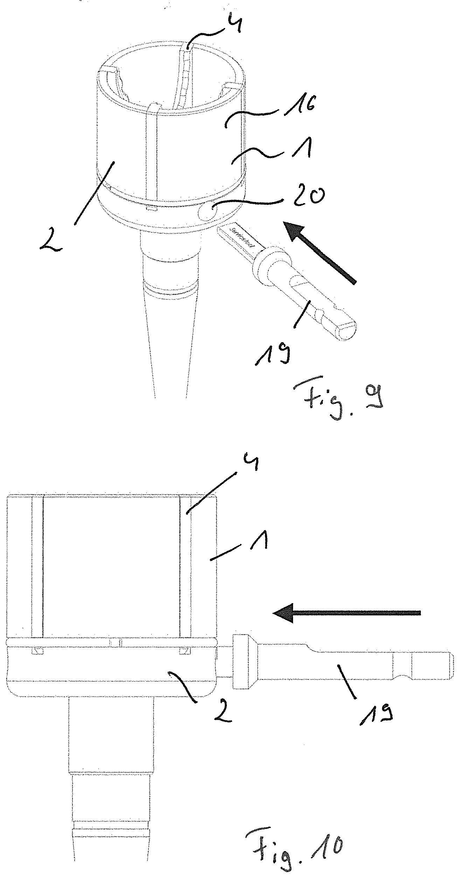

Anhand von Fig. 9 bis 16 wird nun ein bevorzugter Lösevorgang anhand eins Systems, umfassend einen Knochenfräser 1 und ein Lösewerkzeug 19 beschrieben.

Zu erkennen ist eine seitliche im Querschnitt kreisringförmig konturierte Aufnahme 20 in der Umfangswand 16 des Fräsmesserhalters 2, in welche das Lösewerkzeug 19 in die dargestellte Pfeilrichtung einführbar ist. Das Lösewerkzeug 19 kann somit unterhalb des Fräsmessers 4 geführt werden. Das Lösewerkzeug 19 wird bis zum Anschlag eingeführt, wobei der Einführvorgang detaillierter in Fig. 10 ersichtlich ist.

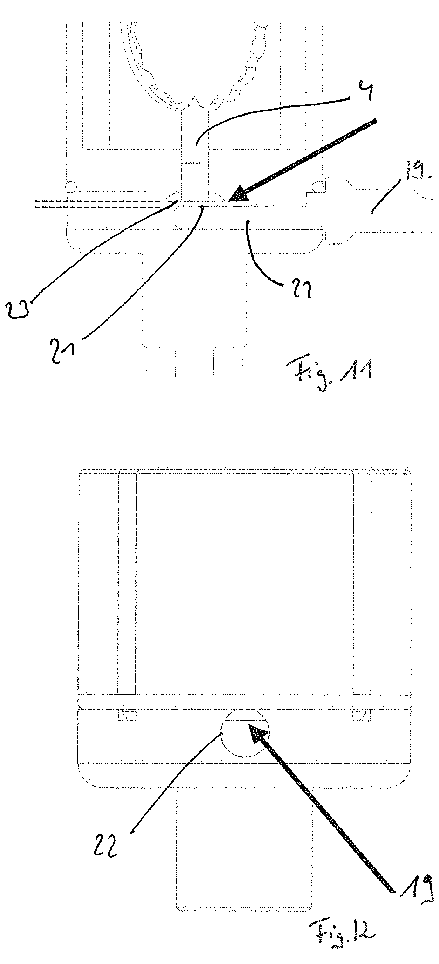

Aus der Schnittansicht gemäß Fig. 11 sowie der Seitenansicht gemäß Fig. 12 ist ersichtlich, dass ein Einführabschnitt 22 des Lösewerkzeugs 12 abgeflacht ausgebildet ist, so dass eine Oberseite 21 zunächst axial beabstandet ist von einer Unterseite des in die Aufnahme 20 (Aufnahmekanal) axial hineinragenden Fräsmessers 4. Aus der Seitenansicht kann man gut erkennen, dass der Einführabschnitt 22 querschnittlich die Form eines angeschnittenen Zylinders aufweist, wobei der untere Vollmaterialabschnitt für eine formkongruente Aufnahme und Führung am Innenumfang der Aufnahme 22 Sorge trägt.

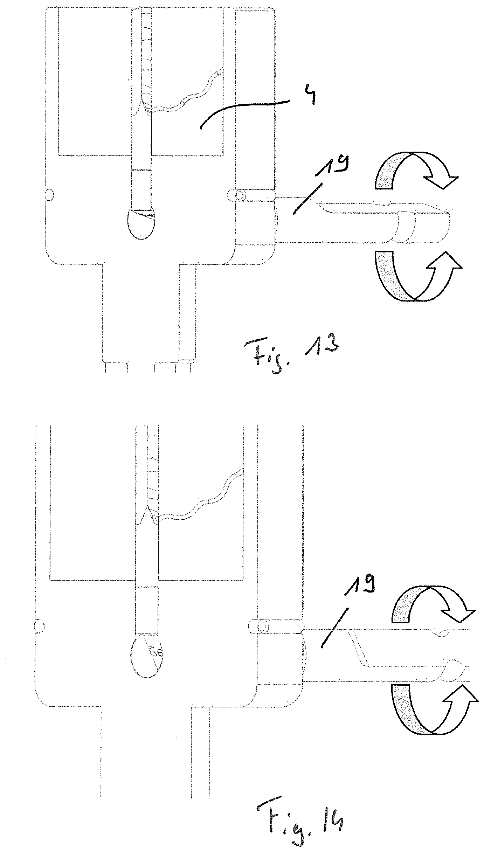

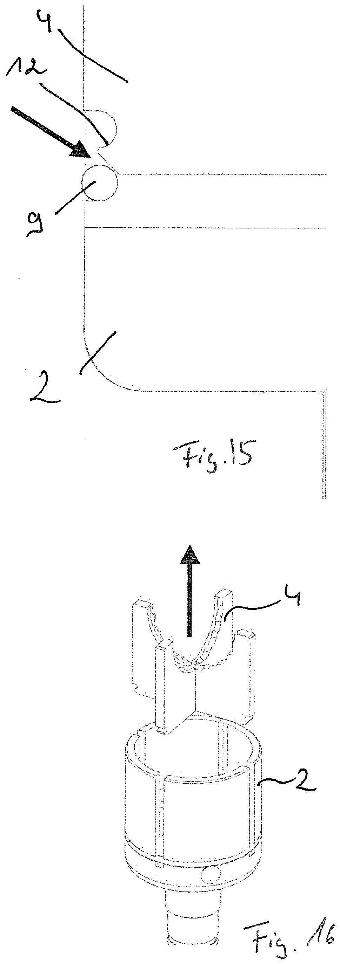

Wie aus den Fig. 13 und 14 ersichtlich ist, kommt das Lösewerkzeug 19 durch Verdrehen um seine Längserstreckungsachse in Kontakt mit dem Fräsmesser 4 und beaufschlagt dieses in axiale Richtung nach oben, wodurch aufgrund der Krümmung der Rastausnehmung 12 eine Radialkraftkomponente resultiert auf das Federelement 9, dieses wieder zeitweise beim Lösevorgang in radialer Richtung nach außen kraftbeaufschlagt und gespannt wird und so das Fräsmesser 4 aus der Verrastung freikommt, wie dies in Fig. 15 gezeigt ist. Dort befindet sich das Federelement wieder im entspannten bzw. entspannteren Zustand und das freie Fräsmesser 4 kann wie in Fig. 16 gezeigt axial entnommen werden.

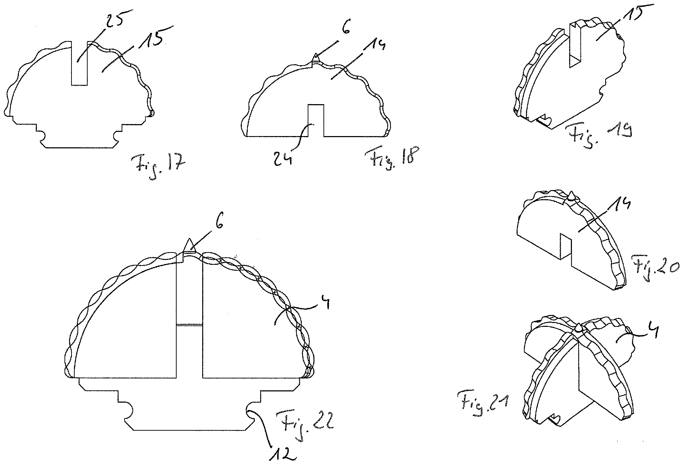

In den Fig. 17 bis 22 sind unterschiedliche Teile und Ausführungen von konvexen Fräsmessern 4 dargestellt. Dabei zeigen Fig. 17 und 19 ein unteres zweites Messerteil 15 und die Fig. 18 und 20 das dazu passende obere (erste) Messerteil 14. Das erste Messerteil 14 weist eine untere Aussparung 24 in der Breite des zweiten Messerteils 15 auf. Im Messerteil 15 ist eine korrespondierende, der Aussparung 24 entgegengerichtete Aussparung 25 in der Breite des ersten Messerteils 14 vorgesehen, so dass die Messerteile 14, 15, wie in Fig. 21 gezeigt und ihren Aussparungen 24, 25 ineinandersteckbar sind und somit eine konvexe Hüllkontur beschreiben. Wie bereits mehrfach erwähnt können die Messerteile 14, 15 lösbar ineinandersteckbar ausgebildet sein oder aneinander, beispielsweise durch Verkleben oder Verschweißen festgelegt oder einteilig hergestellt sein.

In dem gezeigten Ausführungsbeispiel ist das zweite Messerteil 15 gemäß Fig. 17 hinsichtlich seiner Klingenabschnitte asymmetrisch ausgebildet, wohingegen das erste Messerteil gemäß Fig. 18 symmetrisch ausgestaltet ist. Es sind auch Ausführungen realisierbar, bei der beide Messerteile symmetrisch oder beide Messerteile asymmetrisch ausgebildet sind. Fig. 22 zeigt ein Fräsmesser 4, welches aus einem asymmetrischen zweiten und einem symmetrischen ersten Messerteil zusammengesetzt ist.

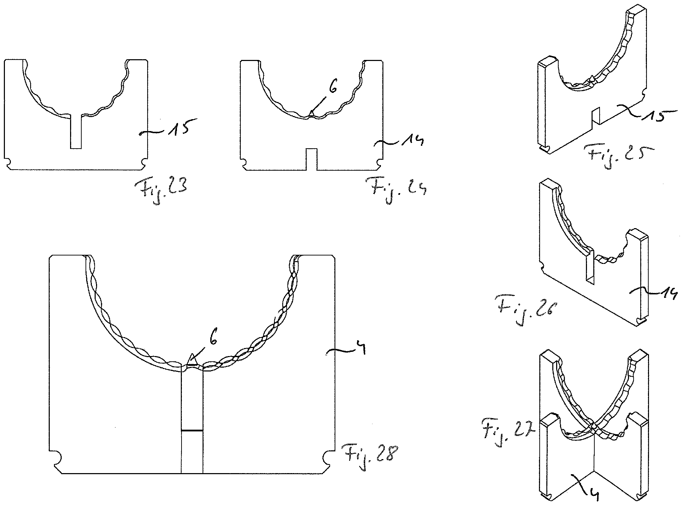

Analog zeigen die Fig. 23 bis 28 unterschiedliche Bauteile bzw. Ausführungsformen eines konkaven Messerteils 4, wobei Fig. 23 und 25 ein zweites Messerteil und Fig. 24 ein dazugehöriges erstes Messerteil 14 zeigen, die in der Art wie das Messerteil gemäß den Fig. 17 bis 22, sich kreuzend zusammensteckbar sind. Das Messerteil gemäß Fig. 23 ist hinsichtlich seiner beiden Klingenabschnitte asymmetrisch ausgebildet, wohingegen das erste Messerteil 14 gemäß Fig. 24 symmetrisch ausgestaltet ist. Fig. 28 zeigt ein aus diesen beiden Messerteilen 14, 15 zusammengesetztes Fräsmesser 4. Sämtliche Fräsmesser gemäß den Fig. 17 bis 28 haben einen Wellenschliff.

### Bezugszeichen

- 1: Knochenfräser
- 2: Fräsmesserhalter
- 3: Abtrieb
- 4: Fräsmesser
- 5a bis 5d: Klingenabschnitte
- 6: Zentrierspitze
- 7: Fixiermittel
- 8: Formschlussmittel
- 9: Federelement
- 10: Umfangsnut
- 11: Axialführungen
- 12: Rastausnehmungen
- 13: Schlitz
- 14: erstes Messerteil
- 15: zweites Messerteil
- 16: Umfangsnut
- 17: Anlaufschrägen
- 18: Anlagefläche
- 19: Lösewerkzeug
- 20: Aufnahme
- 21: Oberseite
- 22: Einführabschnitt
- 23: Unterseite
- 24: Aussparung
- 25: Aussparung

## Patentansprüche

1. Chirurgischer Knochenfräser, insbesondere für die Arthrodese eines, vorzugsweise des ersten, Metatarsophalangealgelenks, mit einem rotierbaren Fräsmesserhalter (2) und mindestens einem Fräsmesser (4) zur Herstellung einer konkaven oder konvexen Fräskontur im Knochen, welches mit Fixiermitteln (7) lösbar an dem Fräsmesserhalter (2) fixierbar ist,
wobei als Fixiermittel (7) ein Federelement (9) umfassende Rastmittel zur lösbaren Verrastung des Fräsmessers (4) am Fräsmesserhalter (2) vorgesehen sind,
wobei das Federelement (9) derart ausgebildet und angeordnet ist, dass dieses beim Festlegen und/oder Lösen des Fräsmessers (4) in radialer Richtung spannbar ist und im verrasteten Zustand in eine Rastausnehmung (12) aufgenommen ist,
wobei das Federelement (9) am Fräsmesserhalter (2), insbesondere in einer Nut des Fräsmesserhalters (2) festgelegt ist und beim Festlegen des Fräsmessers (4) durch unmittelbare Wechselwirkung mit dem Fräsmesser (4) spannbar ist,
wobei das Fräsmesser (4) das Federelement (9) im verrasteten Zustand axial innen durchsetzt oder außen umgreift,
wobei als Federelement (9) mindestens ein, bevorzugt ausschließlich ein, insbesondere geschlitzter Federring und/oder mindestens zwei in Umfangsrichtung beabstandete, teilkreisförmige Federn vorgesehen ist/sind, und wobei Formschlussmittel (8) zum Sichern des Fräsmessers (4) gegen Rotation relativ zu dem Fräsmesserhalter (2) vorgesehen sind, insbesondere in Form mindestens einer Axialführung zur Führung des Fräsmessers (4) beim Festlegen und/oder Lösen desselben,
**dadurch gekennzeichnet,**
**dass** das Federelement die Formschlussmittel (8) in Umfangsrichtung durchsetzt.

2. Knochenfräser nach Anspruch 1,
**dadurch gekennzeichnet,**
**dass** zum radialen Spannen am Fräsmesser (4) eine Anlaufschräge (17) vorgesehen ist.

3. Knochenfräser nach Anspruch 1 oder 2,
**dadurch gekennzeichnet,**
**dass** an dem Fräsmesserhalter (2) eine Werkzeugaufnahme zur Aufnahme (20) eines Lösewerkzeugs (19) vorgesehen ist und dass das Fräsmesser (4) derart in die Werkzeugaufnahme (20), insbesondere senkrecht zu einer Aufnahmelängserstreckung, hineinragt, dass das Fräsmesser (4), insbesondere durch Verdrehen des Lösewerkzeugs (19) in der Aufnahme (20), axial in eine Löserichtung kraftbeaufschlagbar ist.

4. Knochenfräser nach einem der vorhergehenden Ansprüche,
**dadurch gekennzeichnet,**
**dass** das Fräsmesser (4) einteilig ausgebildet ist oder mehrteilig, insbesondere mit mindestens zwei axial ineinandersteckbaren Messerteilen.

5. Knochenfräser nach einem der vorhergehenden Ansprüche,
**dadurch gekennzeichnet,**
**dass** das Fräsmesser (4) einen Wellschliff aufweist.

6. Knochenfräser nach einem der vorhergehenden Ansprüche,
**dadurch gekennzeichnet,**
**dass** das Fräsmeser (4) sich, vorzugsweise rechtwinklig, kreuzende Messerteile (14, 15) aufweist.

7. Knochenfräser nach einem der vorhergehenden Ansprüche,
**dadurch gekennzeichnet,**
**dass** das Fräsmesser (4) eine Zentrierspitze (6) aufweist.

8. Sortiment umfassend einen Knochenfräser (1) nach einem der vorhergehenden Ansprüche mit einem ersten Fräsmesser (4) und mindestens einem zweiten, von dem ersten Fräsmesser (4) unterschiedlichen, vorzugsweise einen unterschiedlichen Durchmesser aufweisenden Fräsmesser (4).

9. System, umfassend einen Knochenfräser (1) nach einem der Ansprüche 1 bis 7 sowie ein Lösewerkzeug (19), das in eine Aufnahme (20) des Fräsmesserhalters (2) einführbar ist, und dass das Fräsmesser (4) im verrasteten Zustand derart in die Aufnahme (20), insbesondere senkrecht zur einer Aufnahmelängserstreckung, hineinragt, dass das Fräsmesser (4) mittels des Lösewerkzeug (19), insbesondere durch Verdrehen des Lösewerkzeugs (19) in der Aufnahme (20), derart axial in eine Löserichtung kraftbeaufschlagbar ist, dass das Fräswerkzeug das Federelement (9) in radialer Richtung spannt, um aus der Verrastung frei zu kommen.

## Claims

1. A surgical bone cutter, in particular for the arthrodesis of a metatarsophalangeal joint, preferably of the first metatarsophalangeal joint, having a rotatable cutter blade holder (2) and having at least one cutter blade (4) for the production of a concave or convex cutting contour in a bone, which cutter blade can be releasably fixed to the cutter blade holder (2) by fixing means (7),
wherein latching means comprising a spring element (9) are provided as fixing means (7) for a releasable latching of the cutter blade (4) to the cutter blade holder (2);
wherein the spring element (9) is configured and arranged such that it can be tensioned in a radial direction on a fixing and/or release of the cutter blade (4) and such that it is received into a latch recess (12) in a latched state;
wherein the spring element (9) is fixed to the cutter blade holder (2), in particular in a groove of the cutter blade holder (2), and can be tensioned by a direct interaction with the cutter blade (4) on the fixing of the cutter blade (4);
wherein the cutter blade (4) penetrates the spring element (9) axially inwardly or engages around it outwardly in the latched state;
wherein at least one, preferably only one, spring ring which is in particular slotted and/or at least two partly circular springs which are spaced apart from one another in the peripheral direction is/are provided as the spring element (9); and
wherein shape-matching means (8) are provided for securing the cutter blade (4) against rotation relative to the cutter blade holder (2), in particular in the form of at least one axial guide for guiding the cutter blade (4) on its fixing and/or release,
**characterized in that**
the spring element penetrates the shape-matching means (8) in the peripheral direction.

2. A bone cutter in accordance with claim 1,
**characterized in that**
a run-on chamfer (17) is provided at the cutter blade (4) for a radial tensioning.

3. A bone cutter in accordance with claim 1 or claim 2,
**characterized in that**
a tool mount (20) for mounting a releasing tool (19) is provided at the cutter blade holder (2); and **in that** the cutter blade (4) projects into the tool mount (20), in particular perpendicular to a longitudinal extent of said mount, such that the cutter blade (4) can be axially acted on by force in a release direction, in particular by a rotating of the releasing tool (19) in the mount (20).

4. A bone cutter in accordance with any one of the preceding claims,
**characterized in that**
the cutter blade (4) is configured in one part or in multiple parts, in particular having at least two cutter parts which can be plugged into one another in an axial manner.

5. A bone cutter in accordance with any one of the preceding claims,
**characterized in that**
the cutter blade (4) has a serration.

6. A bone cutter in accordance with any one of the preceding claims,
**characterized in that**
the cutter blade (4) has cutter parts (14, 15) which intersect one another, preferably at right angles.

7. A bone cutter in accordance with any one of the preceding claims,
**characterized in that**
the cutter blade (4) has a centering tip (6).

8. An assorted set comprising a bone cutter (1) in accordance with any one of the preceding claims having a first cutter blade (4) and having at least one second cutter blade (4) which differs from the first cutter blade (4) and which preferably has a different diameter.

9. A system comprising a bone cutter (1) in accordance with any one of the claims 1 to 7 and a releasing tool (19) which can be introduced into a mount (20) of the cutter blade holder (2), wherein the cutter blade (4) in the latched state projects into the mount (20), in particular perpendicular to a longitudinal extent of said mount, such that the cutter blade (4) can be axially acted on by force in a release direction by means of the releasing tool (19), in particular by a rotating of the releasing tool (19) in the mount (20), such that the cutting tool tensions the spring element (9) in the radial direction in order to be released from the latch connection.

## Revendications

1. Fraise à os chirurgicale, en particulier pour l'arthrodèse d'une articulation de phalange du métatarse, de préférence de la première, comprenant un porte-couteau de fraisage (2) rotatif et au moins un couteau de fraisage (4) pour la réalisation d'un contour de fraisage concave ou convexe dans l'os, qui est susceptible d'être fixé de façon détachable sur le porte-couteau de fraisage (2) avec un moyen de fixation (7),
dans laquelle il est prévu à titre de moyen de fixation (7) un organe d'enclenchement incluant un élément à ressort (9) pour l'enclenchement détachable du couteau de fraisage (4) sur le porte-couteau de fraisage (2),
dans laquelle l'élément à ressort (9) est réalisé et agencé de telle façon que, lors de l'immobilisation et/ou le détachement du couteau de fraisage (4), celui-ci peut être bandé en direction radiale et est reçu, dans l'état enclenché, dans un logement d'enclenchement (12),
dans laquelle l'élément à ressort (9) est immobilisé sur le porte-couteau de fraisage (2), en particulier dans une gorge du porte-couteau de fraisage (2), et, lors de l'immobilisation du couteau de fraisage (4), peut être bandé par interaction directe avec le couteau de fraisage (4),
dans laquelle le couteau de fraisage (4) traverse axialement l'élément à ressort (9) à l'intérieur ou enserre celui-ci de l'extérieur dans l'état enclenché,
dans laquelle il est prévu à titre d'élément à ressort (9) au moins un et de préférence exclusivement un anneau à ressort, en particulier fendu, et/ou au moins deux ressorts de forme circulaire partielle, écartés en direction périphérique, et
dans laquelle il est prévu des moyens à coopération de formes (8) pour bloquer le couteau de fraisage (4) à l'encontre d'une rotation par rapport au porte-couteau de fraisage (2), en particulier sous la forme d'au moins un guidage axial pour le guidage du couteau de fraisage (4) lors de l'immobilisation et/ou du détachement de celui-ci,
**caractérisée en ce que**
l'élément à ressort traverse les moyens à coopération de formes (8) en direction périphérique.

2. Fraise à os selon la revendication 1,
**caractérisée en ce qu'**il est prévu une pente d'attaque (17) sur le couteau de fraisage (4) pour le bandage radial.

3. Fraise à os selon la revendication 1 ou 2,
**caractérisée en ce qu'**il est prévu sur le porte-couteau de fraisage (2) un logement d'outil pour recevoir (20) un outil de libération (19), et **en ce que** le couteau de fraisage (4) pénètre dans le logement d'outil (20), en particulier perpendiculairement à une extension longitudinale du logement, de telle façon que le couteau de fraisage (4) est susceptible d'être attaqué par une force axiale en direction de libération, en particulier par rotation de l'outil de libération (19) dans le logement (20).

4. Fraise à os selon l'une des revendications précédentes,
**caractérisée en ce que** le couteau de fraisage (4) est réalisé d'une seule pièce ou en plusieurs pièces, en particulier avec de parties de couteau susceptible d'être enfichées axialement l'une dans l'autre.

5. Fraise à os selon l'une des revendications précédentes,
**caractérisée en ce que** la fraise à os (4) comporte un meulage ondulé.

6. Fraise à os selon l'une des revendications précédentes,
**caractérisée en ce que** le couteau de fraisage (4) comprend des parties de couteau (14, 15) qui se croisent, de préférence à angle droit.

7. Fraise à os selon l'une des revendications précédentes,
**caractérisée en ce que** le couteau de fraisage (4) comporte une pointe de centrage (6).

8. Assortiment incluant une fraise à os (1) selon l'une des revendications précédentes avec un premier couteau de fraisage (4) et au moins un second couteau de fraisage (4) différent du premier couteau de fraisage (4) et présentant de préférence un diamètre différent.

9. Système, incluant une fraise à os (1) selon l'une des revendications 1 à 7, ainsi qu'un outil de libération (19), qui est susceptible d'être introduit dans un logement (20) du porte-couteau de fraisage (2), et en ce que le couteau de fraisage (4) pénètre, dans l'état enclenché, dans le logement (20), en particulier perpendiculairement à une extension longitudinale du logement, de telle façon que le couteau de fraisage (4) est susceptible d'être attaqué en force au moyen de l'outil de libération (19), en particulier par rotation de l'outil de libération (19) dans le logement (20), axialement dans une direction de libération, de telle façon que l'outil de fraisage bande l'élément à ressort (9) en direction radiale, afin de se dégager de l'enclenchement.
